# EUROPEAN PATENT APPLICATION

(11) **EP 0 729 766 A1**
(43) Date of publication of application: **04.09.1996**
(21) Application number: 95200533.8
(22) Date of filing: 03.03.1995
(51) Int. Cl.: A61M 25/00

(54) **Microcatheter**

(71) Applicant: CORDIS EUROPA N.V., NL-9301 LJ Roden (NL)
(72) Inventor: van Muiden, Johannes Gerardus Maria, NL-9321 AZ Peize (NL)
(74) Representative: 't Jong, Bastiaan Jacobus

(57) **Abstract**

The invention relates to a microcatheter comprising a tube-like basic body with at least one lumen extending from a distal to a proximal end, for which a connecting member has been applied to the proximal end and wherein, in the direction from the proximal towards the distal end, the diameter of the basic body decreases, over at least part of its length, and the flexibility increases, wherein the decrease in diameter and the increase in flexibility are obtained in that a flexible layer of material has a substantially constant thickness over the length of the catheter and the thickness of a stiff layer of material decreases from the proximal towards the distal end.

## Description

The invention relates to a microcatheter specially designed for use in the tortuous, small blood vessels in the human brain. At the distal end such a catheter should be pliable to such an extent that it can pass through very small bends, smaller than 5 mm, in the vascular system of the brain. At the same time the catheter should be sufficiently stiff towards the proximal end to transmit the longitudinal forces when introducing and manipulating the catheter. Furthermore, these qualities must be combined in a catheter of which the distal end must be capable of penetrating into very narrow blood vessels with a diameter considerably smaller than 1 mm.

The microcatheter according to the invention as characterised in claim 1 has the properties with which these aims can be achieved.

During catheterization the catheter is introduced into a relatively wide blood vessel and advanced from there to ever narrower vessels. Towards the proximal end the thickness of the catheter may therefore be greater, without limiting the accessibility of the very small blood vessels. Because of the somewhat greater thickness close to the proximal end, the microcatheter can be manipulated properly.

A desired increase in the pliability towards the distal end is already achieved due to a decrease in the diameter but is additionally enhanced, by using relatively stiff material close to the proximal end and increasingly, rather more pliable material towards the distal end. In that way a desired, great difference in flexibility of the distal end and the proximal end can be achieved.

In order to be able to use the catheter according to the invention with a metal guide wire or one coated with polytetrafluorethylene, which obviously will have a very small diameter, the measure as characterised in claim 3 is preferably employed. The friction between the guide wire and the catheter will consequently be so little that, even in case of a very tortuous passage of the catheter in the vascular system in the brain, the guide wire will not get stuck inside the catheter. The layer of material characterised by low friction can for instance be made of polytetrafluorethylene or polyethylene, which at the same time can withstand aggressive chemotherapeutics or wear from the guide wire.

In order to achieve a good pressure resistance and a good torsional resistance at least close to the proximal end, the measure as characterised in claim 3 is preferably employed.

To reduce axial flexibility at the distal end, claim 4 is employed in an advantageous manner.

The microcatheter according to the invention will have for the intended application preferably the dimensions as characterised in claim 5.

The invention will be explained in greater detail with reference to four examples of embodiments shown schematically in the attached drawings.

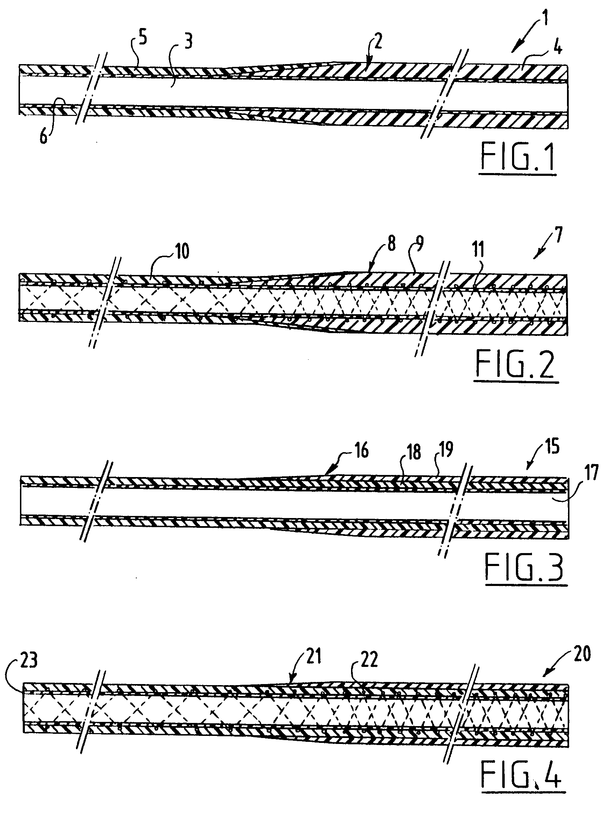

Each of the figures 1 up to and including 4 illustrates schematically a basic body of a catheter according to the invention, with the proximal end on the right hand side and the distal end on the left hand side.

The basic body 2 of the catheter 1 shown in fig. 1 comprises one lumen 3 which runs from the proximal end, to which a connecting member, not shown here, has been attached, to the distal end. As the figure illustrates, the diameter of the basic body 2 decreases from the proximal end towards the distal end.

The basic body 2 is made up of a relatively stiff layer of material 4 and a relatively flexible layer of material 5. The cross-section of the stiff layer of material 4 decreases in relation to the flexible layer of material 5 in a transition zone. This transition zone can have a length of several tens of centimetres, for instance something like 50 cm. Close to the proximal end the stiff layer of material 4 has a constant cross-section and close to the distal end the flexible layer of material 5 has a constant cross-section.

The gradual transition from the one layer of material 4 to the other 5, can be achieved by manufacturing the basic body 2 by intermittent extrusion. The supply of the stiff material 4 is gradually reduced, while the supply of the flexible material 5 is gradually increased. In order to achieve the reduction in diameter, the reduction in the supply of the stiff material 4 will however be set at less than the increase in the supply of the flexible material 5.

The reduction in outside diameter from the proximal towards the distal end can also be achieved by increasing the carrying velocity of the extrusion product during the transition from the stiff to the flexible material.

The lumen 3 is bounded by a layer 6 made of a low friction material, such as polytetrafluorethylene or polyethylene, in order to achieve minimal friction as regards a guide wire to be advanced through the lumen 3.

The catheter 7 as shown in fig. 2 is basically similar to the catheter 1 as shown in fig. 1. Also in this case the basic body 8 comprises one lumen, made of a stiff layer of material 9 and a flexible layer of material 10, wherein towards the distal end the diameter of the basic body 8 decreases.

The catheter 7 comprises furthermore a braided or wound reinforcing layer 11 of metal or plastic threads or filaments. As the figure illustrates, the pitch of the braided or wound reinforcing layer is smaller at the proximal end than at the distal end, but, with another embodiment, one can also opt for the pitch to be constant from the proximal to the distal end of the catheter. In this case the word pitch is understood to mean the distance between the threads or filaments. By using a smaller pitch close to the proximal end, the number of threads per unit length is greater, so that the reinforcing layer contributes more to the torsional resistance of the catheter than at the distal end. At the distal end the reinforcing layer contributes more to the reduction in flexibility due to the larger pitch.

The catheter 15 illustrated in fig. 3 comprises a basic body 16 which has been made slightly different from the catheter 1 in fig. 1. The catheter 15 also comprises one lumen 17, a flexible layer of material 18 and a stiff layer of material 19. However, the thickness of the flexible layer of material 18 is constant over the entire length of the basic body 16 whereas the thickness of the stiff layer of material 19 reduces to zero towards the distal end. Consequently a change in the flexibility and the thickness of the catheter is achieved. The lumen is bounded by a layer with good frictional properties like for instance polytetrafluorethylene or polyethylene.

The catheter 20 of fig. 4 has basically the same construction again as the one in fig. 3, wherein however once again a reinforcing layer 22 has been incorporated in the basic body 21 of which the threads can have an increasing pitch towards the distal end.

In the catheter 20 a layer 23 with good frictional properties has been applied. As has been mentioned before, this layer is preferably a polytetrafluorethylene or polyethylene layer.

For the stiff layer of material a suitable polyurethane or a low pressure polyethylene can be chosen, while for the flexible layer also a polyurethane, but in that case of a composition which displays little stiffness, or a polyethylene with a vinyl acetate can be chosen.

With the invention a microcatheter, suitable for the application as described, of considerable length and a very small diameter of less than 1 mm, can be manufactured. From the proximal end to the distal end the diameter can decrease from basically 1 mm to basically 0,7 mm. The wall thickness of the catheter can in that case be in the order of 0,1 mm, so that the inside diameter of the lumen can be something like 0,5 mm.

## Claims

1. Microcatheter comprising a tube-like basic body with at least one lumen extending from a distal to a proximal end, for which a connecting member has been applied to the proximal end and wherein, in the direction from the proximal towards the distal end, the diameter of the basic body decreases, over at least part of its length, and the flexibility increases, wherein the decrease in diameter and the increase in flexibility are obtained in that a flexible layer of material has a substantially constant thickness over the length of the catheter and the thickness of a stiff layer of material decreases from the proximal towards the distal end.

2. Microcatheter as claimed in claim 1, wherein the lumen is bounded by a layer of low friction material.

3. Microcatheter as claimed in one of the previous claims, comprising a reinforcing layer braided or wound of threads or filaments.

4. Microcatheter as claimed in claim 3, wherein the pitch of the braided or wound threads or filaments increases towards the distal end.

5. Microcatheter as claimed in one of the previous claims, wherein the diameter decreases from 1,0 or 0,8 mm to 0,75 or 0,65 mm.
